# EUROPÄISCHE PATENTANMELDUNG

(11) **EP 0 887 356 A1**
(43) Veröffentlichungstag der Anmeldung: **30.12.1998**
(21) Anmeldenummer: 98108162.3
(22) Anmeldetag: 05.05.1998
(51) Int. Cl.: C08F 8/14, C08F 8/32, C08F 228/02, C08G 18/62, C09D 175/00

(54) **Blutverträgliches und bakterienabweisendes NCO-reaktiv modifiziertes Copolymer**

(30) Priorität: 24.06.1997 DE 19726737
(71) Anmelder: HÜLS AKTIENGESELLSCHAFT, 45764 Marl (DE)
(72) Erfinder: Anders, Christine, Dr., 45721 Haltern (DE); Lorenz, Günter, Dr., 52068 Aachen (DE)

(57) **Zusammenfassung**

Die Erfindung betrifft NCO-reaktiv modifiziertes Copolymer, das (a) mindestens ein sulfonatgruppenhaltiges Monomer, (b) mindestens ein carboxylgruppenhaltiges Monomer und (c) mindestens eine Art von nach der Copolymerisation eingeführten NCO-reaktiven Gruppen enthält. Die Erfindung betrifft auch ein Verfahren zur Herstellung des NCO-reaktiv modifizierten Copolymers, bei dem man mindestens ein sulfonatgruppenhaltiges Monomer (a) und mindestens ein carboxylgruppenhaltiges Monomer (b) radikalisch initiiert zu einem primären Copolymer polymerisiert und dieses mit einer polyfunktionellen NCO-reaktiven Verbindung zu einem NCO-reaktiv modifizierten Copolymer umsetzt, ein Verfahren zur Modifizierung von hydrophilen Oberflächen unter Verwendung des NCO-reaktiv modifizierten Copolymers sowie Erzeugnisse mit modifizierten hydrophilen Oberflächen.

## Beschreibung

Die Erfindung betrifft ein blutverträgliches und bakterienabweisendes NCO-reaktiv modifiziertes Copolymer, ein Verfahren zu dessen Herstellung, ein Verfahren zur Modifizierung von hydrophilen Oberflächen mit diesem Copolymer sowie Erzeugnisse mit derart modifizierten Oberflächen und deren Verwendung für verschiedene, in der Folge erläuterte Zwecke, insbesondere für medizinische Zwecke.

### 1. Stand der Technik

Die Ansiedelung und Vermehrung von Bakterien auf Oberflächen ist eine in der Regel unerwünschte Erscheinung, die häufig mit nachteiligen Folgen verbunden ist. So können in der Trinkwasser- und Getränketechnik Bakterienpopulationen zu einer gesundheitsgefährdenden Qualitätsminderung führen. Bakterien auf oder in Verpackungen bewirken häufig den Verderb von Lebensmitteln oder verursachen sogar Infektionen bei dem Verbraucher. In steril zu betreibenden biotechnischen Anlagen stellen systemfremde Bakterien ein erhebliches prozeßtechnisches Risiko dar. Solche Bakterien können mit Rohstoffen eingetragen werden oder bei mangelhafter Sterilisation in allen Anlageteilen zurückbleiben. Teile der Bakterienpopulation können sich durch Adhäsion dem normalen Flüssigkeitsaustausch beim Spülen und Reinigen entziehen und sich im System vermehren.

Weiterhin sind Bakterienansiedelungen in Wasseraufbereitungsanlagen (z.B. zur Entsalzung durch Membranen) oder auch in Behältern bekannt, die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen,

Besondere Bedeutung kommt dem Schutz vor Bakterienanhaftung und -ausbreitung in der Ernährung, der Pflege, hier insbesondere in der Altenpflege, und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung, z.B. in Bädern. Solche Einrichtungen sind bevorzugte Lebensräume für Bakterien, ebenso wie bestimmte Oberflächen in Bereichen mit hohem Publikumsverkehr so z.B. in öffentlichen Verkehrsmitteln, Krankenhäusern, Telefonzellen, Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und in der häuslichen Pflege.

Besondere Sorgfalt bedarf die Verwendung medizinischer Gegenstände und Geräte bei medizinischen Untersuchungen, Behandlungen und Eingriffen, vor allem dann, wenn derartige Geräte oder Gegenstände mit lebendem Gewebe oder mit Körperflüssigkeiten in Kontakt kommen. Im Falle von Langzeit- oder Dauerkontakten, beispielsweise bei Implantaten, Kathetern, Stents, Herzklappen und Herzschrittmachern, können Bakterienkontaminationen zu einem lebensbedrohenden Risiko für den Patienten werden.

Es wurde bereits auf vielfältige Weise versucht, die Ansiedelung und Ausbreitung von Bakterien auf Oberflächen zu unterbinden. In J. Microbiol. Chemoth. **31** (1993), 261-271 beschreiben S.E.Tebbs und T.S.J.Elliott lackartige Beschichtungen mit quaternären Ammoniumsalzen als antimikrobiell wirkenden Komponenten. Es ist bekannt, daß diese Salze von Wasser, wäßrigen oder anderen polaren Medien sowie von Körperflüssigkeiten aus dem Beschichtungsmaterial herausgelöst werden und ihre Wirkung somit nur von kurzer Dauer ist. Dies gilt gleichermaßen für die Einarbeitung von Silbersalzen in Beschichtungen, so beschrieben in WO 92/18098.

T. Ouchi und Y. Ohya beschreiben in Progr.Polym.Sci. **20** (1995), 211 ff., die Immobilisierung von bakteriziden Wirkstoffen auf Polymeroberflächen durch kovalente Bindung oder ionische Wechselwirkungen. Häufig sind in solchen Fällen die keimtötenden Wirkungen gegenüber dem reinen Wirkstoff deutlich reduziert. Heteropolare Bindungen erweisen sich oft als nicht hinreichend stabil. Darüber hinaus führt die Keimabtötung in der Regel zu unerwünschten Ablagerungen auf den Oberflächen, die die weitere bakterizide Wirkung maskieren und die Grundlage für eine nachfolgende Bakterienbesiedelung bilden.

W. Kohnen et al. berichten in ZBl. Bakt. Suppl. 26, Gustav Fischer Verlag, Stuttgart-Jena-New York, 1994, Seiten 408 bis 410, daß die Adhäsion von Staphylococcus epidermidis auf einem Polyurethanfilm vermindert wird, wenn der Film durch eine Glimmentladung in Gegenwart von Sauerstoff vorbehandelt und dann mit Acrylsäure gepfropft wird.

Bei Gegenständen zur Verwendung für medizinische Zwecke, d.h. für Untersuchungen, Behandlungen und Eingriffe, wie zuvor beschrieben. spielen nicht nur die bakterienabweisenden Eigenschaften eine Rolle, vielmehr kommt es auch auf Blutverträglichkeit, d.h. auf eine möglichst lange Blutgerinnungszeit bzw. möglichst wenig ausgeprägte thrombogene Eigenschaften an. Nach der internationalen Patentanmeldung WO 94/17904 lassen sich Membranen für medizinische Zwecke durch Behandlung mit einem Niederdruckplasma so modifizieren, daß u.a. ihre thrombogenen Eigenschaften gegenüber unbehandelten Membranen herabgesetzt sind. Unter den geeigneten Plasma-bildenden Gasen wird auch Schwefeldioxid aufgeführt. J.-C, Lin et al. beschreiben in Biomaterials **16** (1995), 1017-1023, die Plasma-Behandlung der inneren Oberfläche von LDPE-Rohren, wobei wiederum Schwefeldioxid als plasmabildendes Gas eingesetzt werden kann. Die Autoren berichten, daß die durch SO₂-Plasma modifizierten Oberflächen Sulfonatgruppen enthielten und stark hydrophil, aber in höherem Maße thrombogen waren als die unbehandelten Oberflächen. Die Autoren vermuten, daß dies auf die kombinierte Wirkung von Oberflächenchemie, also der Sulfonierung, und der Hydrophilie der Oberflächen zurückgeht.

Der vorliegenden Erfindung lag die Aufgabe zugrunde, ein nachhaltig blutverträgliches und bakterienabweisendes Copolymer bereitzustellen, das ohne Wirkungsverlust auf der Oberfläche von Substraten, insbesondere von Kunststoffsubstraten durch kovalente chemische Bindungen und daher dauerhaft fixiert werden kann und keine Inaktivierung durch abgetötete Bakterien oder sonstige Ablagerungen zeigt.

### 2. Kurzbeschreibung der Erfindung

Es wurde überraschenderweise gefunden, daß diese Aufgabe gelöst wird durch ein NCO-reaktiv modifiziertes Copolymer, das (a) mindestens ein sulfonatgruppenhaltiges Monomer, (b) mindestens ein carboxylgruppenhaltiges Monomer und (c) mindestens eine Art von nach der Copolymerisation eingeführten NCO-reaktiven Gruppen enthält.

Das molare Verhältnis von Carboxylgruppen zu Sulfonatgruppen, die zusammenwirkend die gewünschten blutverträglichen und bakterienabweisenden Eigenschaften ergeben, beträgt zweckmäßig 0,1 bis 10, vorteilhaft 0,2 bis 10 und insbesondere 0,2 bis 5.

Ein anderer Gegenstand der Erfindung ist ein Verfahren zur Herstellung des NCO-reaktiv modifizierten Copolymers, bei dem man mindestens ein sulfonatgruppenhaltiges Monomer (a) und mindestens ein carboxylgruppenhaltiges Monomer (b) radikalisch initiiert zu einem primären Copolymer polymerisiert und dieses mit einer polyfunktionellen, NCO-reaktiven Verbindung zu einem NCO-reaktiv modifizierten Copolymer umsetzt.

Ein weiterer Gegenstand der Erfindung ist ein Verfahren zur Modifizierung von hydrophilen Oberflächen, insbesondere von Polymersubstraten, bei dem das NCO-reaktiv modifizierte Copolymer durch Umsetzung mit einem Polyisocyanat kovalent auf der Oberfläche gebunden wird.

Schließlich sind ein Gegenstand der Erfindung Erzeugnisse mit derart modifizierten Oberflächen zur Verwendung für hygienische, technische, nahrungsmittel- und biotechnische sowie insbesondere für medizinische Zwecke.

### 3. Vorteile der Erfindung

Das erfindungsgemäße NCO-reaktiv modifizierte Copolymer vermindert die Adhäsion und Vermehrung von Bakterien in einem hohen Maße auch über lange Zeit. Diese Wirkung wird durch die Fixierung über kovalente Bindungen nicht beeinträchtigt. Von dieser Wirkung betroffene Bakterien sind u.a. Staphylococcus aureus, Staphylococcus epidermidis, Streptococcus pyogenes, Klebsiella pneumoniae, Pseudomonas aeruginosa und Escherichia coli. Wenn es insbesondere bei medizinischen Verwendungen darauf ankommt, daß das Copolymer frei von migrationsfähigen Monomeren oder Oligomeren ist, kann man diese mit geeigneten Lösemitteln, wie Wasser oder Wasser/Ethanol-Gemischen, extrahieren. Die physikalischen und chemischen Eigenschaften des Substratmaterials bleiben nach der Oberflächenmodifizierung praktisch unverändert. Unerwünschte Nebenwirkungen durch freigesetzte körperfremde Stoffe oder durch abgetötete Bakterien treten nicht ein.

### 4. Beschreibung der Erfindung

Der Erfindung liegt der Gedanke zugrunde, ein an sich bekanntes, aus üblichen, gut zugänglichen Monomeren aufgebautes Copolymer so zu modifizieren, daß es NCO-reaktiv wird und durch Reaktion mit einem Polyisocyanat kovalent auf einer hydrophilen Oberfläche fixiert werden kann, Zur Herstellung des erfindungsgemäßen NCO-reaktiv modifizierten Copolymers werden also zunächst mindestens ein sulfonatgruppenhaltiges Monomer (a) und mindestens ein carboxylgruppenhaltiges Monomer (b), die olefinisch ungesättigt sind, in einer radikalisch initiierten Reaktion zu einem Copolymer polymerisiert, das als primäres Copolymer bezeichnet wird, weil es in einer zweiten Stufe zum erfindungsgemäßen NCO-reaktiv modifizierten Copolymer umgesetzt wird. Die genannten Monomeren können außer der olefinischen Doppelbindung und der Sulfonat- bzw. Carboxylgruppe weitere funktionelle Gruppen enthalten, die die bioaktiven, die Verarbeitungseigenschaften und/oder die Verträglichkeit des NCO-reaktiven Copolymers mit dem jeweiligen Substrat modifizieren. Weiterhin kann gegebenenfalls mindestens ein weiteres Monomer (d) einpolymerisiert werden, durch das bzw. die wahlweise ebenfalls modifizierende funktionelle Gruppen eingeführt werden können.

### 4.1 Monomere für das primäre Copolymer

Von den geeigneten sulfonatgruppenhaltigen **Monomeren (a)** seien beispielsweise Vinylsulfonate und Styrolsulfonate, wie Natriumvinylsulfonat und insbesondere Natriumstyrolsulfonat (o- und p-Isomere) sowie Natriummethallysulfonat genannt. Man kann auch die entsprechenden olefinisch ungesättigten Sulfonsäuren als Monomere für die Copolymerisation einsetzen und erhält dann Copolymere mit Sulfonsäuregruppen. Solche Copolymere bzw. die daraus hergestellten NCO-reaktiven Copolymere gehören auch zum Bereich der Erfindung. Wenn man die Sulfonsäuregruppen nachträglich neutralisiert, z.B. mit Natriumhydroxid, erhält man Produkte, die den von vornherein mit den entsprechenden Sulfonatmonomeren hergestellten entsprechen.

Geeignete carboxylgruppenhaltige **Monomere (b)** sind z.B. Acrylsäure, Methacrylsäure, 4-Vinylsalicylsäure, Itaconsäure, Vinylessigsäure, Zimtsäure, 4-Vinylbenzoesäure, 2-Vinylbenzoesäure, Methylmaleinsäure, Dihydroxymaleinsäure, Crotonsäure, Isocrotonsäure, Fumarsäure, Methylfumarsäure, Dimethylfumarsäure, Allylessigsäure und insbesondere Maleinsäure. Die für die Polymerisation eingesetzten Monomeren (b) können anstelle der Carboxylgruppen zunächst davon abgeleitete Gruppen enthalten, z.B. Ester-, Amid-, Nitril- oder Anhydridgruppen, die nach der Polymerisation in üblicher Weise in Carboxylgruppen umgewandelt werden. Beispiele hierfür sind (Meth)acrylsäurederivate, wie Methylmethacrylat, Ethylacrylat, Acrylnitril und Acrylamid. Weiterhin kann die Carboxylgruppe im Monomer und im primären Copolymer auch ganz oder teilweise neutralisiert sein, z.B. durch Natriumhydroxid. Solche Carboxylatgruppen gelten als Carboxylgruppen im Sinne dieser Erfindung.

Natürlich kann man anstelle eines einzelnen Monomers (a) oder (b) entsprechende Mischungen einsetzen, z.B. statt Natriumstyrolsulfonat allein eine Mischung aus Natriumstyrolsulfonat und Natriumvinylsulfonat oder statt Maleinsäure allein eine Mischung aus Maleinsäure und Acrylsäure.

Das primäre Copolymer kann ausschließlich aus den Monomeren (a) und (b) bestehen. Beispiele für weitere **Monomere (d)**, die man wahlweise miteinpolymerisieren kann und die funktionelle Gruppen enthalten können, sind u.a. Vinylketone, wie Vinylmethylketon und Vinylbutylketon; vinylaromatische Monomere, wie Styrol und Vinyltoluol, α-Methylstyrol und Vinyltoluol; Olefine, wie Ethylen, Propylen, 1-Buten und 1-Octen; Diolefine, wie Butadien und Isopren; Vinylester, wie Vinylacetat und Vinylpropionat; sowie N-Vinylpyrrolidon. Die Monomeren (d) werden gegebenenfalls in der Regel in Mengen von bis zu 30 Gewichtsprozent, bezogen auf die Summe der Monomeren (a), (b) und (d), eingesetzt.

### 4.2 Herstellung des primären Copolymers

Die Monomeren (a), (b) und gegebenenfalls (d) können im primären und im erfindungsgemäßen NCO-reaktiv modifizierten Copolymer, je nach Reaktionsführung bei der Polymerisation, als Blöcke oder statistisch verteilt vorliegen, Die Sulfonat- und die Carboxylgruppe ergeben die erwünschten blutverträglichen und bakterienabweisenden Eigenschaften des erfindungsgemäß NCO-reaktiv modifizierten Copolymers, während dessen NCO-reaktiven Gruppen die kovalente Fixierung (oder Anbindung) des Copolymers auf der hydrophilen Oberfläche des Substrats durch Reaktion mit einem Polyisocyanat ermöglichen.

Das primäre Copolymer wird in üblicher Weise durch radikalisch initiierte Polymerisation der Monomeren (a), (b) und gegebenenfalls (d) hergestellt, vorteilhaft durch Lösungs- oder Emulsionspolymerisation. Ein gut geeignetes Lösemittel ist Wasser, in dem die Monomeren und auch das Copolymer in der Regel gut löslich sind. Gut geeignet sind auch stark polare organische Lösemittel, wie Dimethylformamid, Dimethylacetamid und Dimethylsulfoxid. Zu den anderen geeigneten Lösemitteln zählen Tetrahydrofuran (THF) und Aceton. Ein geeignetes Lösemittel für eine gegebene Kombination von Monomeren läßt sich durch orientierende Versuche unschwer finden.

Geeignete Polymerisationsinitiatoren sind u.a. Azonitrile, Alkylperoxide, Acylperoxide, Hydroperoxide, Peroxyketone, Peroxyester und Percarbonate sowie alle üblichen Photoinitiatoren.

Die Polymerisation wird thermisch, z.B. durch Erhitzen auf 60 bis 100°C oder durch Strahlung mit entsprechender Wellenlänge initiiert. Man kann z.B. einen Teil der Monomeren (a) und (b) sowie gegebenenfalls (d) vorlegen, die Polymerisation starten und die restlichen Monomeren als Gemisch in den Reaktor einführen, wobei man durch Kühlen zweckmäßig eine Temperatur von 60 bis 100°C aufrechterhält. Auf diese Weise erhält man ein statistisches Copolymer. Wenn man ein bestimmtes Monomer oder einen Anteil davon vorlegt und das bzw. die andere(n) Monomer(e) sowie gegebenenfalls die Restmenge des vorgelegten Monomers jeweils für sich portionsweise zugibt, entsteht ein Block-Copolymer. Natürlich sind diese Varianten idealtypische Grenzfälle. In der Praxis erhält man schon infolge der unterschiedlichen Reaktionsgeschwindigkeit der verschiedenen Monomeren neben Bereichen mit weitgehend statistischer Verteilung auch solche mit überwiegender Blockstruktur.

Nach Beendigung der Polymerisation erhält man, je nach den Anteilen der Monomeren (a), (b) sowie gegebenenfalls (d) und dem verwendeten Lösemittel, eine Lösung oder Emulsion des primären Copolymers mit Feststoffgehalten, die z.B. 5 bis 20 Gew.-% betragen können.

### 4.3 NCO-reaktive Modifizierung des primären Copolymers

Das primäre Copolymer aus den Monomeren (a), (b) und gegebenenfalls (d) wird in einem zweiten Reaktionsschritt NCO-reaktiv modifiziert, indem es mit einer polyfunktionellen NCO-reaktiven Verbindung umgesetzt wird, Dadurch wird mindestens eine Art von NCO-reaktiven Gruppen in das primäre Copolymer eingeführt bzw. zusätzlich eingeführt, zu denen insbesondere Hydroxyl- und primäre oder sekundäre Aminogruppen gehören. Diese Gruppen sind zugleich Carboxyl-reaktiv und reagieren bei der NCO-reaktiven Modifizierung mit einer Carboxylgruppe des primären Copolymers unter Bildung einer Estergruppe bzw. einer Carbonamidgruppe. Als polyfunktionelle NCO-reaktive Verbindungen eignen sich vorzugsweise Polyole, wie Diole oder Triole: Aminoalkohole, wie Amino- oder N-Alkylaminoalkanole; sowie Polyamine, wie Diamine und Triamine. Als Beispiele seien genannt: Ethylenglykol, Propylenglykol, 1,4-Butandiol, 1.6-Hexandiol, Trimethylolpropan, Aminoethanol, N-Methylaminoethanol, Ethylendiamin, Propylendiamin, Hexamethylendiamin und Diethylentriamin. Als Ergebnis der Umsetzung erhält man ein NCO-reaktiv modifiziertes primäres Copolymer, in dem die NCO-reaktiven Gruppen in Seitenketten angeordnet sind, welche über Carbonester- oder Carbonamidgruppen an das aus Kohlenstoffatomen bestehende Gerüst des primären Copolymers gebunden sind.

Die NCO-reaktiven Gruppen ermöglichen die kovalente Fixierung (oder Anbindung) des Copolymers auf der hydrophilen Oberfläche des Substrats durch Reaktion mit einem verknüpfenden Polyisocyanat. Der Anteil der NCO-reaktiven Gruppen an der Gesamtfunktionalität des modifizierten primären Copolymers (d.h. Sulfonat-, Carboxyl- und NCO-reaktive Gruppen) beträgt zweckmäßig etwa 5 bis 20 Molprozent. Ein solcher molarer Anteil ermöglicht und gewährleistet eine feste Anbindung auf hydrophilen Oberflächen mit Hilfe eines Polyisocyanats.

Da bei der Umsetzung des primären Copolymers mit der polyfunktionellen NCO-reaktiven Verbindung zum NCO-reaktiven Copolymer, wie zuvor erläutert, Carboxylgruppen verbraucht werden, muß der molare Anteil der Carboxylgruppen an der Gesamtfunktionalität des primären Copolymers (d.h. Sulfonat- plus Carboxylgruppen) entsprechend höher sein, wenn ein bestimmtes Verhältnis von Carboxyl- und Sulfonatgruppen und ein bestimmter molarer Anteil an NCO-reaktiven Gruppen an der Gesamtfunktionalität des NCO-reaktiven Copolymers vorgegeben ist. Die Menge der bei dieser Umsetzung eingesetzten polyfunktionellen NCO-reaktiven Verbindung hängt von deren Funktionalität (bi-, trifunktionell), dem molaren Anteil der Carboxylgruppen im primären Copolymer und dem gewünschten molaren Verhältnis von Carboxyl- und Sulfonatgruppen im erfindungsgemäßen NCO-reaktiv modifizierten Copolymer ab.

Zur NCO-reaktiven Modifizierung kann das primäre Copolymer in einem Autoklaven mit einem Aminoalkohol, z.B. Ethanolamin, oder einem Diamin, z.B. Hexamethylendiamin, bei erhöhter Temperatur und erhöhtem Druck umgesetzt werden. Der Verlauf der Umsetzung kann mittels potentiometrischer Titration oder NMR-Spektroskopie verfolgt werden. Alternativ kann die Modifizierung auch drucklos vorgenommen werden, indem man das primäre Copolymer und die modifizierende Komponente in einem hochsiedenden Lösemittel erhitzt, zweckmäßig in Gegenwart eines Schleppmittels für Wasser.

### 5. Verwendung des modifizierten Copolymers als Beschichtungsmittel

Eine Lösung oder Emulsion des NCO-reaktiv modifizierten Copolymers kann zur Modifizierung von hydrophilen Oberflächen, insbesondere von hydrophilen Kunststoffoberflächen verwendet werden. Dabei wird das Copolymer mittels eines Polyisocyanats, in der Regel eines Diisocyanats, durch kovalente Bindungen auf der Oberfläche des hydrophilen Substrats fixiert. Hierfür eignen sich die bekannten handelsüblichen Polyisocyanate, wie Hexamethylendiisocyanat (HMDI). Toluylendiisocyanat (TDI), 4,4'-Methylenbis(phenylisocyanat) (MDI) 4,4'-Methylenbis(cyclohexylisocyanat) (H-MDI) und Isophorondiisocyanat (IPDI). Das Polyisocyanat reagiert mit den Hydroxyl- oder Aminogruppen des Copolymers und mit Hydroxyl- oder Aminogruppen, die auf der hydrophilen Oberfläche des Substrats vorhanden sind, unter Ausbildung von Urethan- oder Harnstoffbrücken. Natürlich werden manche Polyisocyanat-Moleküle ausschließlich mit Hydroxyl- oder Aminogruppen des NCO-reaktiv modifizierten Copolymers unter Kettenverlängerung oder -verknüpfung reagieren. Bei den zuvor genannten molaren Anteilen der NCO-reaktiven Gruppen an der Gesamtfunktionalität des erfindungsgemäßen NCO-reaktiv modifizierten Copolymers findet aber immer in hinreichendem Maße die erwünschte Fixierung auf der Substratoberfläche statt.

Überraschenderweise hat sich herausgestellt, daß die Mitverwendung eines Kettenverlängerungsmittels bei der Anbindung des erfindungsgemäßen NCO-reaktiv modifizierten Copolymers zu einer - mittels ESCA nachweisbaren - höheren Konzentratrion an bioaktiven Gruppen auf dem Substrat und damit zu einer verstärkten Wirkung führt. Kettenverlängerungsmittel sind Verbindungen mit in der Regel endständigen NCO-reaktiven Gruppen, wie Hydroxyl- oder Aminogruppen. Gut geeignet sind z.B. Diamine, Aminoalkohole und Diole, insbesondere Polyalkylenglykole, mit (gegebenenfalls mittleren) Molgewichten von etwa 100 bis 3.000.

Wenn das NCO-reaktiv modifizierte Copolymer in einem wäßrigen oder anderen mit NCO-Gruppen reaktiven Medium gelöst oder emulgiert vorliegt, müssen die Isocyanatgruppen des Polyisocyanats zunächst blockiert (oder maskiert) werden. Dies geschieht in üblicher Weise mit Verbindungen, die sich bei niedrigen Temperaturen, wie 20 bis 60°C, an die NCO-Gruppen anlagern und bei höheren Temperaturen wieder abspalten. Solche Verbindungen sind z.B. Methylethylketoxim und ε-Caprolactam. Ein gut geeignetes Polyisocyanat zur Verwendung in wäßrigen Systemen ist beispielsweise mit Methylethylketoxim blockiertes Isophorondiisocyanat (IPDI). Liegt das Copolymer in einem nichtwäßrigen und mit NCO-Gruppen nicht reaktiven Medium gelöst oder emulgiert vor, z.B. in Dimethylformamid, so kann man mit einem unblokkierten Polyisocyanat arbeiten, z.B. wiederum mit IPDI oder mit Hexamethylendiisocyanat (HDI), 4,4'-Methylenbis(phenylisocyanat) (MDI), 4,4'-Methylenbis(cyclohexylisocyanat (H-MDI) oder Toluylendiisocyanat (TDI).

Die Kunststoffsubstrate, deren Oberflächen mit dem erfindungsgemäßen Copolymer modifiziert werden, können Homo- oder Copolymere sein, die ihrer Natur nach hydrophil oder hydrophiliert sind, wie in der Folge erläutert, d.h. auf der Oberfläche Hydroxylgruppen und/oder Aminogruppen tragen. Hydrophile und hydrophilierte (Co)polymere werden hier gemeinsam als hydrophil bezeichnet. Zu den im Prinzip geeigneten Basis(co)polymeren zählen z.B. Polyolefine, wie Polyethylen, Polypropylen, Polyisobutylen, Polybutadien, Polyisopren, natürliche Kautschuke und Polyethylen-co-propylen; halogenhaltige Polymere, wie Polyvinylchlorid, Polyvinylidenchlorid, Polychloropren, Polytetrafluorethylen und Polyvinylidenfluorid; Polymere und Copolymere aus vinylaromatischen Monomeren, wie Polystyrol, Polyvinyltoluol, Polystyrol-co-vinyltoluol, Polystyrol-co-acrylnitril, Polystyrol-co-butadien-co-acrylnitril: Polykondensate, beispielsweise Polyester, wie Polyethylenterephthalat und Polybutylenterephthalat; Polyamide, wie Polycaprolactam, Polylaurinlactam und das Polykondensat aus Adipinsäure und Hexamethylendiamin; Polyetherblockamide, z.B. aus Laurinlactam oder Caprolactam und Polyethylenglykol mit durchschnittlich 8, 12 oder 16 Ethoxygruppen; weiterhin Polyurethane, Polyether, Polycarbonate, Polysulfone, Polyetherketone, Polyesteramide und imide, Polyacrylnitril, Polyacrylate und -methacrylate.

Soweit die Polymeren oder Copolymeren nicht hinreichend hydrophil sind, müssen sie hydrophiliert werden. Das ist dann der Fall wenn der Kontaktwinkel von Wasser bei 25°C, gemessen nach dem Verfahren von R.J.Good et al., Techniques of Measuring Contact Angles in Surface and Colloid Sciences, Vol. 11, Plenum Press New York, N.Y., 1979, <30° beträgt. Selbst wenn diese Bedingung erfüllt ist, verbessert eine zusätzliche Hydrophilierung die Haftung des aufgebrachten NCO-reaktiven Polymeren. Für die Hydrophilierung steht eine ganze Reihe von Methoden zur Verfügung. So kann man hydroxylgruppenhaltige Monomere, wie Hydroxyethyl(meth)acrylat oder Hydroxybutyl(meth)acrylat, strahleninduziert auf eine polymere Substratoberfläche aufpfropfen (deutsche Patentanmeldung 197 15 449.2). Statt Monomere zu verwenden, kann man auch ein hydroxylgruppenhaltiges Copolymer auf die Substratoberfläche pfropfen (deutsche Patentanmeldung 197 00 081.9) Weiterhin kann man derartige Copolymere in üblicher Weise auf die Substratoberfläche aufbringen, z.B. durch Spritzen, Tauchen oder Spin-Coating mit Lösungen der Copolymeren. Weiterhin lassen sich nicht hinreichend hydrophile Polymere oder Copolymere durch Behandlung mit Argon-Plasma oder Bestrahlung mit UV-Strahlen von 100 bis 400 nm hydrophilieren. Durch Behandlung mit Ammoniak-Plasma erreicht man nicht nur eine Hydrophilierung, sondern durch Einführung von Aminogruppen zusätzliche Bindungsmöglichkeiten sowie physiologische Effekte. Schließlich führt auch eine Ätzung mit starken Säuren, wie Schwefelsäure, Salzsäure und Salpetersäure, oder Basen, wie Alkalimetallhydroxiden, zu einer hinreichenden Hydrophilierung von hydrophoben Polymeren oder Copolymeren.

Die hydrophilen oder hydrophilierten Substratoberflächen werden mit der Lösung oder Emulsion des erfindungsgemäßen NCO-reaktiv modifizierten Copolymers, die ein gegebenenfalls blockiertes Polyisocyanat enthält, in üblicher Weise beschichtet, z.B. durch Tauchen, Spritzen oder Spin-Coating. Nach dem Verdampfen des Lösemittels und gegebenenfalls nach Abspaltung des Blockierungsmittels findet bei 120 bis 150°C die Fixierung des Copolymers auf der Substratoberfläche statt. Man hält diese Temperatur zweckmäßig 10 sec bis 5 Minuten aufrecht und erhält dann eine chemisch gebundene Beschichtung, die nicht selten eher kohäsiv (d.h. in sich) als adhäsiv (d.h. an den Grenzflächen) versagt. Die Beschichtung läßt sich nicht mit Lösemitteln selektiv vom Kunststoffsubstrat ablösen, was einer kovalente Bindung auf dessen Oberfläche entspricht.

Bei einer Variante des Beschichtungsverfahrens behandelt man zunächst die hydrophile oder hydrophilierte Substratoberfläche mit dem gegebenenfalls blockierten Polyisocyanat, um das Polyisocyanat mittels einer seiner NCO-Gruppen auf der Oberfläche zu fixieren, und bringt dann auf die vorbehandelte Fläche die Lösung oder Emulsion des NCO-reaktiv modifizierten Copolymers auf, dessen Hydroxyl- oder Aminogruppen mit den verbliebenen NCO-Gruppen des Polyisocyanats reagieren.

### 6. Erfindungsgemäße Erzeugnisse

Erzeugnisse, wie Vorrichtungen, Geräte und andere Gegenstände, mit erfindungsgemäß, ganz oder teilweise, modifizierten Oberflächen eignen sich für viele Verwendungen, bei denen es darauf ankommt, Bakterienadhäsion und - wachstum zu vermeiden oder zurückzudrängen. Sie sind also u.a. für hygienische, technische, nahrungsmittel- und biotechnische Zwecke geeignet, wie sie beispielhaft zuvor beschrieben wurden. Die Gegenstände eignen sich vorzugsweise für medizinische Verwendungen, besonders dann wenn es gleichzeitig auf bakterienabweisende Eigenschaften und Blutverträglichkeit ankommt. Aber auch bei Kontakten mit anderen Körperflüssigkeiten als Blut, wie Lymphe, oder mit Gewebe kommen die vorteilhaften Eigenschaften der erfindungsgemäßen Gegenstände zur Geltung. Medizinische Verwendungen sind z.B. solche als Lymphdrainagen, Wundverbände, Tubusse, Stents, Herzklappen, Membranen, Blutbeutel oder als Katheter, z.B. bei der Peritonealdialyse.

Die folgenden Beispiele sollen die Erfindung weiter erläutern, nicht aber ihren Umfang begrenzen, wie er in den Patentansprüchen dargelegt ist.

### Beispiele 1 bis 4

### Herstellung des primären Copolymers

200 ml einer 1 M Lösung von Maleinsäure in Wasser werden mit 200 ml einer 1 M Lösung von Natriumstyrolsulfonat in Wasser gemischt. Das Reaktionsgemisch wird mit 1 Mol-%, bezogen auf die Gesamtmenge der Monomeren, Kaliumperoxidisulfat als Starter versehen und 15 Minuten mit Stickstoff durchströmt. Der Ansatz wird auf 60°C erhitzt und 4 Stunden unter Stickstoffatmosphäre gerührt. Anschließend wird das Reaktionsgemisch in Ethanol eingerührt, wobei das Copolymer ausfällt.

### Polymeranaloge Umsetzung des primären Copolymers zu NCO-reaktiven Copolymeren

### (i) Mit Ethanolamin im Autoklaven (Beispiele 1 bis 3)

In einem 200 ml Autoklaven werden jeweils 60g des primären Copolymers mit den in Tabelle 1 angegebenen Komponenten und unter den dort angegebenen Bedingungen umgesetzt. Die Umsatzbestimmung erfolgte durch potentiometrische Bestimmung der Restsäure.

**Tabelle 1**

| | Beisp. 1 | Beisp. 2 | Beisp. 3 |
|---|---|---|---|
| Säureeinheiten des Copolymers (mmol) | 2,768 | 2,768 | 2,768 |
| NaOH (mmol) | 1,384 | - | - |
| Ethanolamin (mmol) | 1,384 | 2,768 | 2,768 |
| Wasser (g) | 29,9 | 38,0 | 36,0 |
| Reaktionszeit (h) | 10 | 10 | 10 |
| Temperatur (°C) | 140 | 180 | 180 |
| Druck (bar) | 3,7 | 9,9 | 9,9 |
| Umsatz (%) | 29,8 | 79,4 | 92,2 |

### (ii) Mit Ethylenglykol unter Normaldruck (Beispiel 4)

60 g des Copolymers werden in 600 ml Ethylenglykol und 600 ml Ethanol (als Schleppmittel für das Reaktionswasser) gelöst. Man erhitzt das Gemisch unter Rühren 6 Stunden auf 100°C. Nach jeweils 0,5 Stunden wird das abdestillierte Ethanol/Wasser-Gemisch mit Ethanol wieder aufgefüllt (insgesamt 250 ml). Anschließend werden die flüchtigen Anteile bei vermindertem Druck (20 mbar) und einer Temperatur bis zu 120°C abdestilliert. Der Rückstand wird zweimal mit Aceton gewaschen und getrocknet. Der Umsatz beträgt nach Karl-Fischer-Titration 85%.

### Modifizierung von hydrophilen Polymeroberflächen mit NCO-reaktiven Copolymeren mittels Polyisococyanaten

Zur Fixierung der NCO-reaktiven Copolymeren aus den Beispielen 1 bis 4 auf verschiedenen Polymersubstraten werden die Polyisocyanatkomponenten und die NCO-reaktiven Copolymeren in einem geeigneten Lösemittel gelöst und durch Tauchen auf die Oberfläche des Polymersubstrats aufgebracht. Anschließend wird das Lösemittel verdampft und das NCO-reaktive Copolymer durch Erhitzen auf dem Polymersubstrat fixiert. Zur Entfernung von Restmonomeren und von Oligomeren wird das beschichtete Substrat mit Wasser von 60°C gewaschen,

Mittels ESCA-Messungen wird die Beschichtung nachgewiesen. Sie ist anhand der veränderten Elementenzusammensetzung im Vergleich zu den unbeschichteten Polymersubstraten und insbesondere am hinzugekommenen Schwefel erkennbar.

### Bestimmung der primären Bakterienadhäsion unter statischen Bedingungen

Eine Über-Nacht-Kultur des Bakterienstammes Klebsiella pneumoniae in Hefeextrakt-Pepton-Glukose-Nährmedium (1% + 1% + 1%) wird abzentrifugiert und in Phosphat-gepufferter Saline (=PBS; 0,05m KH₂PO₄, pH 7,2 + 0,9 % NaCl) wieder aufgenommen. Man verdünnt mit PBS-Puffer auf ein Zellkonzentration von 10⁸ Zellen/ml. Die suspendierten Bakterien werden mit dem zu untersuchenden Folienstück für 3h in Berührung gebracht. Dazu werden doppelseitig beschichtete kreisförmige Folienstücke mit einem Durchmesser von 1,6 cm (=4.02 cm²) auf eine Präpariernadel gesteckt und mit der Zellsuspension geschüttelt. Einseitig beschichtete Folien werden in Form einer runden, ebenen Scheibe von 4,5 cm Durchmesser und mit einer Stützmembran aus 2-3 cm dickem Weich-PVC in eine Membranfilterapparatur eingespannt. Auf die nach oben zeigende Seite mit der zu prüfenden Beschichtung wird die Zellsuspension aufgegeben und 3h geschüttelt. Die Membranfilterapparatur muß dicht sein, d.h. es darf keine Zellsuspension durch undichte Zellen ausfließen.

Nach Ablauf der Kontaktzeit wird die Bakteriensuspension mit einer Wasserstrahlpumpe abgesaugt, und die Folienstücke werden zum Waschen mit 20 ml steriler PBS-Lösung in einem 100 ml Becherglas 2 min geschüttelt. Das Folienstück wird nochmals in sterile PBS-Lösung eingetaucht und dann in 10 ml erhitztem TRIS/EDTA (0,1M Trishydroxyethylaminomethan, 4 mM Ethylendiamintetraessigsäure, mit HCl auf pH 7,8 eingestellt) für 2 min im siedenden Wasserbad extrahiert.

Mit der Extraktionslösung werden kleine Eppendorf-Cups befüllt und sofort bis zur Biolumineszenz-Bestimmung des extrahierten Adenosintriphosphats (ATP) bei -20°C eingefroren. Die Bestimmung wird wie folgt ausgeführt: In ein transparentes Röhrchen aus Polycarbonat wird 100 µl Reagentienmix (Biolumineszenz-Test CLS II. Fa. BOEHRINGER MANNHEIM GmbH) gegeben, und über einen Zeitraum von 10 sec werden in einem Lichtimpuls-Meßgerät LUMAT LB9501 (Laboratorien Prof. Berthold GmbH, 75323 Bad Wildbad, Deutschland) die Lichtimpulse integriert. Dann wird eine 100 µl Probe zugegeben und erneut gemessen. Die relativen Lichteinheiten (RLU) werden durch Subtraktion der Lichtimpulse im Reagentienmix von der Anzahl der gemessenen Lichtimpulse im kompletten Ansatz erhalten. Dieser Wert steht in Relation zu der Anzahl der an der Folie adhärierten Bakterien. Der Umrechnungsfaktor zwischen dem RLU-Wert und der Bakterienzahl wird bestimmt, indem ein Aliquot von 0,1 ml der Bakteriensuspension mit 10⁸ Zellen/ml in 10 ml heißem TRIS/EDTA extrahiert und dann der ATP-Gehalt bestimmt wird.

In der folgenden Tabelle 2 sind die Bedingungen für die Beschichtung der verschiedenen Polymersubstrate mit den NCO-reaktiv modifizierten Copolymeren sowie die Ergebnisse der Messungen der primären Bakterienadhäsion zusammengestellt. Man erkennt, daß die primäre Bakterienadhäsion auf den erfindungsgemäß beschichteten Polymersubstraten um rund eine Größenordnung geringer ist als auf den unbeschichteten Substraten.

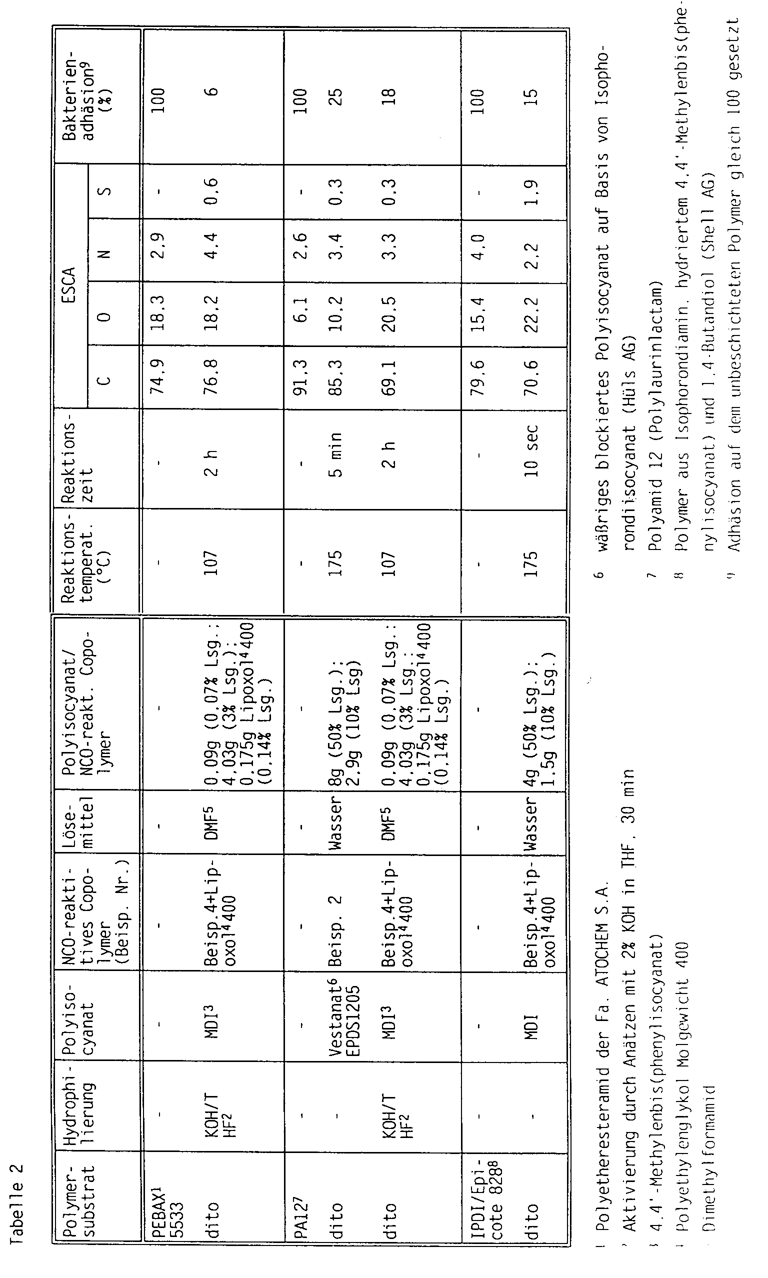

## Patentansprüche

1. NCO-reaktiv modifiziertes Copolymer, dadurch gekennzeichnet, daß es (a) mindestens ein sulfonatgruppenhaltiges Monomer, (b) mindestens ein carboxylgruppenhaltiges Monomer und (c) mindestens eine Art von nach der Copolymerisation eingeführten NCO-reaktiven Gruppen enthält.

2. NCO-reaktiv modifiziertes Copolymer nach Anspruch 1, dadurch gekennzeichnet, daß das sulfonatgruppenhaltige Monomer (a) ein Styrolsulfonat oder ein Vinylsulfonat ist,

3. NCO-reaktiv modifiziertes Copolymer nach Anspruch 1 oder 2, dadurch gekennzeichnet, daß das carboxylgruppenhaltige Monomer (b) Acrylsäure, Methacrylsäure oder Maleinsäure ist.

4. NCO-reaktiv modifiziertes Copolymer nach einem der Ansprüche 1 bis 3, dadurch gekennzeichnet, daß die NCO-reaktiven Gruppen Hydroxyl- oder primäre oder sekundäre Aminogruppen sind.

5. NCO-reaktiv modifiziertes Copolymer nach einem der Ansprüche 1 bis 4, dadurch gekennzeichnet, daß es neben den Monomeren (a) und (b) mindestens ein weiteres Monomer (d) enthält, welches gegebenenfalls funktionelle Gruppen aufweist.

6. NCO-reaktiv modifiziertes Copolymer nach einem der Ansprüche 1 bis 5, dadurch gekennzeichnet, daß das Molverhältnis von Carboxylgruppen zu Sulfonatgruppen 0,1 bis 10 beträgt.

7. NCO-reaktiv modifiziertes Copolymer nach einem der Ansprüche 1 bis 6, dadurch gekennzeichnet, daß der Anteil der NCO-reaktiven Gruppen an der Gesamtfunktionalität des NCO-reaktiv modifizierten Copolymers 5 bis 20 Mol-% beträgt.

8. Verfahren zur Herstellung des NCO-reaktiv modifizierten Copolymers nach einem der Ansprüche 1 bis 7, dadurch gekennzeichnet, daß man mindestens ein sulfonatgruppenhaltiges Monomer (a) und mindestens ein carboxylgruppenhaltiges Monomer (b) radikalisch initiiert zu einem primären Copolymer polymerisiert und dieses mit einer polyfunktionellen NCO-reaktiven Verbindung zu einem NCO-reaktiv modifizierten Copolymer umsetzt.

9. Verfahren nach Anspruch 8, dadurch gekennzeichnet, daß die polyfunktionelle NCO-reaktive Verbindung ein Polyol, Aminoalkohol oder Polyamin ist.

10. Verfahren zur Modifizierung von hydrophilen Oberflächen, insbesondere von hydrophilen Kunststoffoberflächen, bei dem das NCO-reaktiv modifizierte Copolymer nach einem der Ansprüche 1 bis 7 durch Umsetzung mit einem Polyisocyanat kovalent auf der Oberfläche gebunden wird.

11. Verfahren nach Anspruch 10, dadurch gekennzeichnet, daß die Umsetzung in einem wäßrigem Medium stattfindet und das Polyisocyanat ein blockiertes Polyisocyanat ist.

12. Verfahren nach Anspruch 10, dadurch gekennzeichnet, daß die Umsetzung in einem nichtwäßrigem, mit NCO-Gruppen nicht reaktiven Medium stattfindet und das Polyisocyanat ein unblockiertes Polyisocyanat ist.

13. Erzeugnisse mit ganz oder teilweise gemäß einem Verfahren nach einem der Ansprüche 10 bis 12 modifizierter Oberfläche zur medizinischen Verwendung.

14. Erzeugnisse nach Anspruch 13, dadurch gekennzeichnet, daß die Erzeugnisse Lymphdrainagen, Wundverbände, Tubusse, Stents, Herklappen, Membranen, Blutbeutel oder Katheter sind.
